# EUROPEAN PATENT SPECIFICATION

(11) **EP 1 861 130 B1**
(45) Date of publication and mention of the grant of the patent: **10.09.2008**
(21) Application number: 06708228.9
(22) Date of filing: 13.02.2006
(51) Int. Cl.: A61L 15/44, A61K 31/185, A61P 31/10

(54) **DEVICE AND METHOD FOR TREATMENT OF DERMATOMYCOSIS, AND IN PARTICULAR ONYCHOMYCOSIS**
VORRICHTUNG UND VERFAHREN ZUR BEHANDLUNG VON DERMATOMYKOSEN UND INSBESONDERE ONYCHOMYKOSEN
DISPOSITIF ET TECHNIQUE DE TRAITEMENT DE MYCOSE DERMATOLOGIQUE ET EN PARTICULIER D'ONYCHOMYCOSE

(30) Priority: 11.02.2005 EP 05002936; 14.02.2005 US 652759 P; 23.08.2005 EP 05018269; 24.08.2005 US 711006 P
(43) Date of publication of application: 05.12.2007
(73) Proprietor: NOLabs AB, 252 21 Helsingborg (SE)
(72) Inventor: PETERS, Tor, 8200 Schaffhausen (CH)
(74) Representative: Krahbichler, Erik
(86) International application number: PCT/EP2006/050889
(87) International publication number: WO 2006/084910

(56) References cited:
- EP-A- 1 300 424
- WO-A-20/04012874
- WO-A-20/05003032
- WO-A2-01/26702
- US-A- 5 814 666
- US-A1- 2002 082 221
- US-A1- 2002 136 750
- US-A1- 2004 043 068
- SMITH D J ET AL: "Transdermal delivery of nitric oxide from diazeniumdiolates" JOURNAL OF CONTROLLED RELEASE, ELSEVIER, AMSTERDAM, NL, vol. 51, no. 2-3, 12 February 1998 (1998-02-12), pages 153-159, XP004113622 ISSN: 0168-3659
- PULFER S K ET AL: "INCORPORATION OF NITRIC OXIDE-RELEASING CROSSLINKED POLYETHYLENEIMINE MICROSPHERES INTO VASCULAR GRAFTS" JOURNAL OF BIOMEDICAL MATERIALS RESEARCH, WILEY, NEW YORK, NY, US, vol. 37, no. 2, November 1997 (1997-11), pages 182-189, XP000978327 ISSN: 0021-9304

## Description

### Field of the Invention

This invention pertains in general to the field of treatment of dermatomycosis, and in particular onychomycosis and dermatophytosis. More particularly the invention relates to a device for the therapeutic treatment of dermatomycosis of humans and animals, and in particular onychomycosis and dermatophytosis, and a process for manufacturing of said device, involving the use of nitric oxide (NO).

### Background of the Invention

Dermatophytes and yeast fungus are the most common reason for superficial fungal infections, and belong to the few infections that are obtained through direct skin-to-skin contact. They are keratinophilic and infect skin, hair, and nails. In some rare cases mould fungus may be the cause of infection (Most often caused by the species *Fusarium, Scytalidium, Hendersonula Toruloidea, Scopulariopsis Brevicaulis, Aspargillus Nidulans, Acremonium, Exophalia and Alterneria*). Infections from dermatophytes exist all around the world, but are more common in developing countries, since socioeconomic factors and contact with animals play a big role in the transmittance.

Superficial fungal infections are mainly caused by the dermatophytes *Trichophyton, Epidermophyton* and *Microsporum.* These species are categorised into anthropophilic, i.e. transmits through direct or indirect between humans, zoophilic, i.e. transmits from animals to humans, and geophilic, i.e. transmits from soil.

Among the yeast funguses *Candida Albicans* is the most pathogenic. Other important Candida species, that may cause superficial infections are *C. glabrata, C. tropicalis, C. krusei,* and *C*. *parapsilosis.*

Infections from yeast funguses are in most cases caused by *C. Albicans,* and includes vaginitis, stomatitis, dermatitis, paronychia, dermatophytosis (athletes foot) and onychomycosis.

Up to this point different types of antimycotics, such as azoles, terbinafine, amorolfine, nystatine, ciclopiroxolamine etc, are available for the local treatment of infections caused by dermatophytes, yeast fungus, and mould fungus. The different types of antimycotics differentiate somewhat in respect of antimicrobial spectrum and pharmacology. These antimyocotics are suitable for cutaneous dermatophyte infections, and in some extent for mild forms of onychomycosis.

However, these antimycotics do not seldomly cause adverse side effects. Mostly, these adverse side effects are expressed in form of local skin irritation, contact allergic reactions, allergic reactions against preservatives in the antimycotics, drug resistance against the antimycotics etc.

Another way of treating infections from dermatophytes, yeast fungus, and mould fungus is by peroral treatment. Examples of peroral pharmaceuticals are flukanazol, for treatment of dermatological treatment, ketokonazol, for treatment of mucotane candidiasis, itrakonazol, for treatment of infections in nails and skin, and terbinafin, for treatment of infections in nails and skin when local treatment has not given a satisfying result.

Peroral treatment also presents a number of adverse side effects, such as negative symptoms in the gastrointestinal tract, headache, oedema, taste disorders etc. In same rare cases the persons treated perorally have died.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible.

Other example for NO eluting polymers are given in US-5,770,645, wherein polymers derivatized with at least one -NOx group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

US 2002/0082221 discloses a nitric oxide releasing S-nitrosylated, N-nitrosylated, and/or O-nitrosylated lipid and administration methods thereof. This lipid may be integrated and provided within a polymer matrix. Thus, it is not the polymer that elutes NO in US 2002/0082221, but the lipid. Therefore, the system according to US 2002/0082221 is in need of a nitric oxide eluting lipid. Nothing is mentioned of regulating and/or controlling the elution of NO.

US 2002/0136750 discloses a dosage form for the treatment of bacterial, virus, or fungal conditions, said dosage form comprising an acidifying agent and a source of nitrate ions or a precursor thereof, wherein said acidifying agent and nitrate ions are kept separate in carriers. These carriers, comprising acidifying agent and nitrate ions, respectively, are then mixed to induce elution of nitric oxide. Nothing is mentioned of regulating and/or controlling the elution of NO.

EP 1 300 424 discloses extremely hydrophobic NO releasing polymers. These polymers are extensively crosslinked polyamine-derivatized divinylbenzene diazeniumdiolates. Since the polymer according to EP 1 300 424 is extremely hydrophobic, and "highly resistant to penetration by water and insoluble therein", page 9, line 30, it is unclear how the NO is released. Nothing is mentioned of regulating and/or controlling the elution of NO.

US 5,814,666 discloses compositions capable of releasing nitric oxide for the treatment of microorganism-related diseases. The compositions comprise one or more nitric oxide generators, preferably encapsulated in vesicles, such as liposomes. The active moiety of the compositions in US 5,814,666 is N₂O₂⁻. This group may be bound to a polymer. However, nothing is mentioned of regulating and/or controlling the elution of NO.

US 2004/0043068 discloses a medical device coated with a coating, comprising a polyurea network, which can be associated with and release nitric oxide, in one embodiment of US 2004/0043068. US 2004/0043068 does not mention dermal treatment but only vascular diseases and Raynard's disease (see page 9, paragraph 86). Nothing is mentioned of regulating and/or controlling the elution of NO.

WO 2005/003032 discloses zeolites containing releasably adsorbed nitric oxide. Zeolites are not polymers. Nothing is mentioned of regulating and/or controlling the elution of NO.

WO 2004/012874 discloses a nitric oxide releasing medical device. The device comprises a substrate to which an amine-functionalized silane residue can be bound, such as a metallic surface, and nitric oxide bound to the substrate through NO-releasing nucleophiles, which are bonded to said amine-functionalized silane residue. Nothing is mentioned of regulating and/or controlling the elution of NO.

US 6,737,447 discloses a coating for medical devices, which coating provides NO delivery by using nanofibres of L-PEI. Nothing is mentioned of regulating and/or controlling the elution of NO or treatment of dermatomycosis.

Furthermore, the disclosures are silent concerning an improvement of present technology in respect of treatment of disorders caused by dermatophytes, yeast fungus, and mould fungus, and the anti pathogenic potential of nitric oxide.

Hence, an improved, or more advantageous, device for the treatment and/or prevention of infection, caused by dermatophytes, yeast fungus, and mould fungus, such as onychomycosis and dermatophytosis. It is desired that said device does not develop resistance against the active pharmaceutical substance, and which does not cause local skin irritation or contact allergic reactions, negative symptoms in the gastrointestinal tract, headache, oedema, taste disorders etc, would be advantageous, and in particular a device allowing for target prevention and treatment of infections, such as onychomycosis and dermatophytosis, would be advantageous.

### Summary of the Invention

Accordingly, the present invention preferably seeks to mitigate, alleviate or eliminate one or more of the above-identified deficiencies in the art and disadvantages singly or in any combination and solves, among others, at least the problems mentioned above, by providing a device, a manufacturing method for the latter and a use of nitric oxide according to the appended patent claims.

According to one aspect of the invention, a device is provided that allows for target treatment of infections, caused by dermatophytes, yeast fungus, and mould fungus, such as onychomycosis and dermatophytosis. The device comprises a nitric oxide (NO) eluting polymer configured to elute a therapeutic dosage of nitrogen oxide (NO) when used for said treatment, and
wherein said nitric oxide (NO) eluting polymer is integrated with a carrier material, such that said carrier material, in use, regulates and controls the elution of said therapeutic dosage of nitric oxide (NO),
wherein said device is configured to expose a treatment site of said infection, in or on a body, to said nitric oxide when said polymer in use elutes nitrogen oxide (NO), and wherein said elution of nitric oxide (NO) from said device in use substantially is directed towards said target site for said exposure.

According to another aspect of the invention, a manufacturing process for such a device is provided, wherein the process is a process for forming a device that allows for target treatment of infections, caused by dermatophytes, yeast fungus, and mould fungus, such as onychomycosis and dermatophytosis. The process comprises selecting a plurality of nitric oxide eluting polymeric particles, such as nano fibres, fibres, nano particles, or microspeheres, and deploying said nitric oxide eluting particles in a condom/sheath or tape/coating to be comprised in said device. Alternatively the NO eluting particles are admixed to an ointment, cream, gel or foam.

According to another aspect of the invention, a nitric oxide (NO) eluting polymer for use in the treatment of dermatomychosis, including onychomycosis and dermatophytosis, is provided wherein
nitric oxide is loaded to a device so said device elutes nitric oxide (NO) from said eluting polymer in a therapeutic dose when used at a site of dermatomychosis on a body towards said site.

The present invention has at least the advantage over the prior art that it provides target exposure of an infected area to NO, whereby a very effective anti-dermatophyte, anti-yeast fungus, and/or anti-mould fungus therapy is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a condom/sheath according to the invention,
Fig. 2 is a schematic illustration of a tape or coating according to the invention,
Fig. 3 is a schematic illustration of a sock according to the invention.

### Description of Embodiments

The following description focuses on embodiments of the present invention applicable to a device, in form of a condom/sheath, which allows for target treatment of infections caused by dermatophytes, yeast fungus, and mould fungus, such as onychomycosis and dermatophytosis.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible. Another advantage is that NO is released without any secondary products that could lead to undesired side effects.

The polymers may be manufactured by electro spinning, gas spinning, air spinning, wet spinning, dry spinning, melt spinning, or gel spinning. Electro spinning is a process by which a dissolved polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

However, the meaning of "controlled" in the context of US 6,737,447 is only directed to the fact that nitric oxide is eluted from the coating during a period of time, i.e that the nitric oxide not is eluted all in once. Therefore, the interpretation of "controlled" in respect of US 6,737,447 is different from the meaning of "regulating" in the present invention. "Regulate or control", according to the present invention is intended to be interpreted as the possibility to vary the elution of nitric oxide to thereby achieve different elution profiles.

A polymer comprising an O-nitrosylated group is also a possible nitric oxide eluting polymer. Thus, in one embodiment of the present invention, the nitric oxide eluting polymer comprises diazeniumdiolate groups, S-nitrosylated and O-nitrosylated groups, or any combinations thereof.

In still another embodiment of the present invention said nitric oxide eluting polymer is a poly(alkyleneimine)diazeniumdiolate, such as L-PEI-NO (linear poly(ethyleneimine)diazeniumdiolate), where said nitric oxide eluting polymer is loaded with nitric oxide through the diazeniumdiolate groups and arranged to release nitric oxide at a treatment site.

Some other examples of a suitable nitric oxide eluting polymer are selected from the group comprising amino cellulose, amino dextrans, chitosan, aminated chitosan, polyethyleneimine, PEI-cellulose, polypropyleneimine, polybutyleneimine, polyurethane, poly(buthanediol spermate), poly(iminocarbonate), polypeptide, Carboxy Methyl Cellulose (CMC), polystyrene, poly(vinyl chloride), and polydimethylsiloxane, or any combinations of these, and these mentioned polymers grafted to an inert backbone, such as a polysaccharide backbone or cellulosic backbone.

In still another embodiment of the present invention the nitric oxide eluting polymer may be a O-derivatized NONOate. This kind of polymer often needs an enzymatic reaction to release nitric oxide.

Other ways of describing polymers, which may be suitable as nitric oxide eluting polymer, is polymers comprising secondary amine groups (=N-H), such as L-PEI, or have a secondary amine (=N-H) as a pendant, such as aminocellulose.

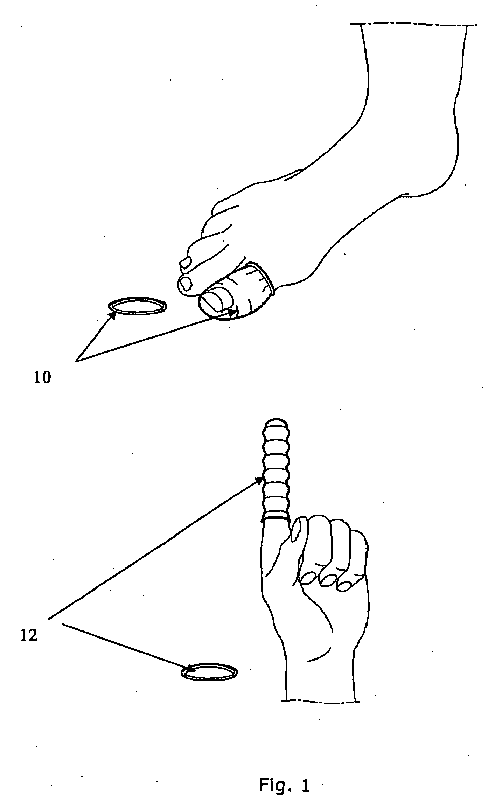

In an embodiment of the invention, according to Fig. 1, the device is in form of a latex or rubber condom/sheath 10, 12, said condom/sheath being covered on the inside with nano-filament of any of the NO-eluting polymers according to above, such as polyalkyleneimines, such as L-PEI (Linear PolyEthyleneImine) and B-PEI (Branched PolyEthyleneImine), which polymers have the advantage of being biocompatible, after the release of nitrogen oxide.

In another embodiment of the present invention the condom/sheath is covered on the inside with nano-filament of L-PEI.

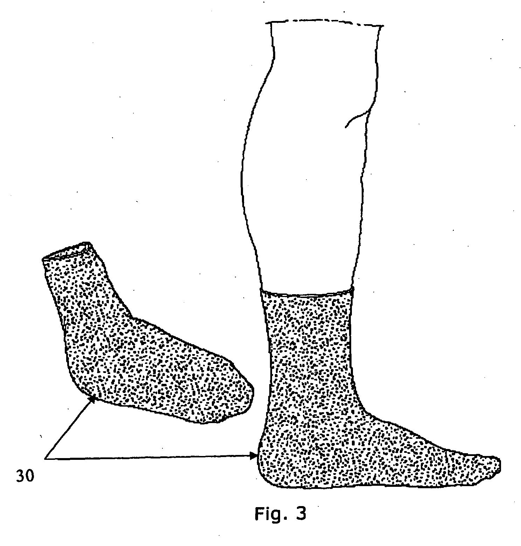

This condom/sheath may be in any suitable size, such as a suitable size for rolling said condom/sheath over the toe or finger, on which toe or finger the nail to be treated is located. These sizes may for example vary from small, medium, and large sized condoms/sheaths for a little finger, ring finger, middle finger, fore finger, or thumb, or small, medium, and large sized condoms/sheaths for a little toe, the three middle toes, or big toe. The condom/sheath according to the invention may even have a size suitable for covering a foot, such as a sock 30, according to Fig. 3, or a foot-condom/sheath, or other specific part of the body, to be able to treat dermatomycosis on larger areas. According to an embodiment, the condoms/sheaths are coated with NO eluting nano fibres. According to another embodiment the condoms/sheaths are made of or comprise nanofilaments, e.g. made by electro or gas jet spinning. Other manufacturing methods, such as wet spinning, dry spinning, melt spinning, and gel spinning, are also within the scope of the present invention. According to a further embodiment the condoms/sheaths comprises microspheres eluting NO in use. Preferably the three aforementioned embodiments employ L-PEI material loaded with NO. Activation on NO release may be done by e.g. foot sweat, water sprayed onto the condoms/sheaths immediately prior to use, or a water bag configured for releasing water upon activation, e.g. by pushing onto the bag thus bursting (see below).

When the NO-eluting condom/sheath according to certain embodiments of the present invention is treated with or gets in contact with the moisture, in form of secreted sweat, the NO-eluting condom/sheath starts to release NO to the area to be treated. Alternatively the device is moistured or wettened immediately prior to application or use for controlling or activating the NO release.

In another embodiment of the present invention a condom/sheath is covered on the inside with NO-eluting nano-particles, or micro-spheres. These nano-particles, or micro-spheres, may be formed from the NO-eluting polymers comprised in the present invention. They may also be encapsulated in any suitable material, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these. When the nano-particles, or micro-spheres, according to this embodiment, gets in contact with the secreted moisture, in form of sweat, on the inside of the condom/sheath, they start to elute NO on the area to be treated.

In the context of the present invention the term "encapsulating" is intended to be interpreted as fixating the nitric oxide eluting polymer in a three dimensional matrix such as a foam, a film, a nonwoven mat of nano-fibers or fibers, or other materials with the capability to fixate the NO eluting polymer, or enclosing the nitric oxide eluting polymer in any suitable material.

In yet another embodiment of the present invention the condom/sheath contains a small proton donor bag or sealed proton donor sponge. This proton donor bag or sealed proton donor sponge is used to activate the elution of NO from the NO-eluting nano-particles, or micro-spheres. This proton donor bag or sealed proton donor sponge may be located in the tip of the condom/sheath according to the invention. Persons that not easily sweat may be helped by the use of this embodiment.

In another embodiment of the present invention a nitric oxide eluting polymer is provided, and/or combined, with microencapsulated proton donor (which will be described in further detail below), such as water or water containing liquid.

This may for example be done by first manufacture micro capsules, containing proton donor, such as water or water containing liquid, in a state of the art manner. These micro capsules are then applied on the NO eluting polymer. The application of the micro capsules on the NO eluting polymer may for example be done by gluing, such as pattern gluing, or instead spinning the NO eluting polymer onto said micro capsules. In this way a device or a system, comprising NO eluting polymer and micro encapsulated water or water containing liquid is manufactured. When the device or system is applied on the target area the device or system is compressed or squeezed. Said compression or squeezing results in breakage of the micro capsules. The NO eluting polymer is thus exposed to said water or water containing liquid, and the elution of NO from the NO eluting polymer is initiated on the target area. In other embodiments of the present invention the liquid inside the micro capsules is released by heating or shearing the micro capsules until the micro capsules are ruptured.

In still another embodiment the micro capsules, are formed into a film, tape, or sheath. Thereafter, a film, tape, or sheath of an NO eluting polymer is glued onto the film, tape, or sheath of micro capsules. Preferably the film, tape, or sheath of the NO eluting polymer is glued onto the film, tape, or sheath of the micro capsules, in patterned way. The obtained pattern includes spaces where there is no glue, in which spaces the proton donor will be transported to the NO eluting polymer once the micro capsules are broken from compression or squeezing. When the proton donor gets in contact with the NO eluting polymer the elution of NO starts. Thus, the combination of film, tape, or sheath of micro capsules, and NO eluting polymer may be applied on a target area. Thereafter the combination is compressed or squeezed, which results in that the target area is exposed to NO.

In yet another embodiment the NO eluting polymer is spun directly onto the film, tape, or sheath of micro capsules, containing proton donor. The combination of film, tape, or sheath of micro capsules, and spun NO eluting polymer may be applied on a target area. Thereafter the combination is compressed or squeezed, which results in that the target area is exposed to NO.

In still another embodiment of the present invention the device or system is provided with an activation indicator. This activation indicator indicates when the micro capsules are satisfyingly broken, hence when the NO eluting polymer is subjected to enough proton donor to elute an efficient amount of NO. This activation indicator may for example be obtained by colouring the proton donor that is trapped inside the micro capsules. When the micro capsules are broken the coloured proton donor escapes the microcapsules and the colour gets visualised while efficiently wetting the NO eluting polymer. Another way of obtaining an activation indicator is to choose to manufacture the micro capsules in a material, or choose a wall thickness of said micro particles, that creates a sound when the micro capsules break. It is also possible to admix a scent in the proton donor, contained in the micro capsules. This results in that the user of the device or system may smell the scent when the proton donor escapes from the micro capsules after breakage thereof.

In another embodiment a substance that changes color when it comes in contact with water can be incorporated in the device. Thus when the water capsules or water bag breaks the material changes color, thereby indicating that the material is activated.

In the present invention the device or system only allows NO-elution in one direction. In this kind of embodiment one side of the device according to the invention has low permeability, or substantially no permeability, to nitric oxide. This may be accomplished by applying a material on one side of the device according to the invention that is not permeable to NO. Such materials may be chosen from the group comprising common plastics, such as fluoropolymers, polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these. This embodiment is also easy to manufacture as the NO eluting polymer, e.g. L-PEI (or nitric oxide eluting polymer and carrier material, which will be explained in more detail below) may be electro or gas-jet spun onto the surface of the device according to the invention of e.g. the mentioned plastics, latex, or cotton.

In still another embodiment the device is provided with one membrane, which is permeable to nitric oxide, on a first side of the device, and another membrane, which has low permeability or substantially no permeability to nitric oxide, on a second side of said device. This embodiment provides the possibility to direct the elution to said first of the device, while the elution of nitric oxide is substantially prevented from said second side. Thereby, a greater amount of nitric oxide will reach the intended area to be treated.

The activation of the nitric oxide eluting polymer may be accomplished by contacting said polymer with a suitable proton donor (as mentioned above). In one embodiment the proton donor may be selected from the group comprising water, body fluids (blood, lymph, bile, etc.), alcohols (methanol, ethanol, propanols, buthanols, pentanols, hexanols, phenols, naphtols, polyols, etc.), aqueous acidic buffers (phosphates, succinates, carbonates, acetates, formats, propionates, butyrates, fatty acids, amino acids, etc.), or any combinations of these.

By adding a surfactant in the proton donor one can facilitate the wettening of the device. The surfactant lowers the surface tension and the activating fluid is easily transported throughout the device.

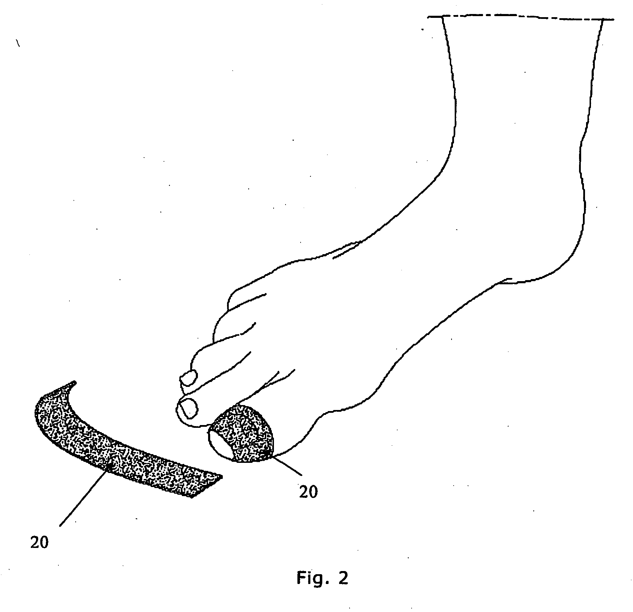

In still another embodiment the device may be manufactured in the form of a polyurethane, or polyethylene, tape or coating 20, according to Fig. 2. This polyurethane tape or coating may easily be wrapped around the toe or finger, at which toe or finger the nail to be treated is located. At least the side facing the toe, or nail, may be covered with NO-eluting nano-particles, or micro-spheres, or nano-filament of NO-eluting L-PEI. When these particles or filaments get in contact with the moisture, in form of sweat, on the inside of the tape or coating, the elution of NO starts.

In another embodiment of the device according to the present invention, it is in form of a patch/pad, which patch/pad is suitable to be applied between the toes or fingers, and onto other areas that are difficult to get at.

Certain embodiments of the invention directly implement treatment by releasing NO to the toe/finger-nail. NO diffuses through the nail and treatment is performed even under the nail. Conventionally, if an infection, or onychomychosis or dermatomychosis, is present under such a nail, the nail is surgically removed and then therapeutic treatment is started. Hence, these embodiments save a patient from a lot of pain and other complications that may occur at during and after these toe removal operations.

Of course, in other embodiments of the invention, the patch/pad or tape/coating may be manufactured by any other suitable material, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these. The NO-eluting polymer may be integrated in, spun together with, or spun on top of, any of these materials in all of the embodiments of the present invention.

In another embodiment these nano-particles, or micro-spheres, may be integrated in a soluble film that disintegrates on the inside of the condom/sheath or tape/coating, in order to elute NO at the area of interest when the soluble film gets in contact with the moisture, in form of sweat or from the water bag or sealed water sponge, on the area to be treated.

When placed on an area to be treated the device provides prevention and treatment of infections, caused by dermatophytes, yeast fungus, and mould fungus, such as onychomycosis and dermatophytosis.

In another embodiment of the present invention the device only allows NO-elution in one direction. In this kind of embodiment one side of the condom/sheath or tape/coating is non-permeable to NO. This may be accomplished by applying a material on one side of the condom/sheath or tape/coating that is not permeable to NO. Such materials may be chosen from the group comprising common plastics, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these. This embodiment is also easy to manufacture as the NO eluting polymer, e.g. L-PEI nano fibres may be electro or gas-jet spun onto the surface of a condom sheath of e.g. the mentioned plastics, latex, or cotton. Other manufacturing methods are also within the scope of the present invention, such as wet spinning, dry spinning, melt spinning, and gel spinning. In the case of a condom it may be rolled up, or a sheath may be turned outside in after manufacturing to protect the NO eluting polymer during packaging, transport and prior to use from external influences, being e.g. mechanical (abrasion of the polymer), chemical (moisture deactivating the device prior to use) etc.

In yet another embodiment of the present invention the NO-eluting device is acting as a booster for drug eluting patches, e.g. pharmaceuticals, vitamins, nicotin, nitroglycerin etc. This embodiment presents a device with the advantage of combining two therapeutic treatments, of significant value, in one treatment. Hence, a synergetic effect may be achieved by such devices when NO that is eluted from the device. NO has a vasodilatory effect on the region where the device having the combination compound actuates. Vasodilated tissue is more susceptible to certain medications and thus more easily treated by the medical preparations and still NO has in addition to that the anti-inflamatory, anti-bacterial etc. effect. Hence, an unexpected surprisingly effective treatment is provided.

In still another embodiment the nitric oxide eluting polymer, such as powder, nano-particles or micro-spheres, can be incorporated in foam. The foam may have an open cell structure, which facilitates the transport of the proton donor to the nitric oxide eluting polymer. The foam can be of any suitable polymer such as polyurethane, polystyrene, polyester, polyvinylchloride, polyolefins, or latex.

In another embodiment the device is in form of a cream, a gel or a combination of the two. Since the nitric oxide eluting polymer is activated by proton donors the nitric oxide eluting polymer has to be separate from the proton donor until one wants to initiate the elution of nitric oxide, i.e. use the device. One way to accomplish this is to have a syringe with two separate containers. In one container you have a proton donor-based gel and in the other a non proton donor-based gel, comprising the nitric oxide eluting polymer. Upon using the device the two gels are squeezed from the syringe and mixed together, the proton donor in the first gel comes in contact with the nitric oxide eluting polymer in the second gel and the elution of nitric oxide starts.. The elution of NO may then be initiated by applying a water soaked patch on said gel or foam. This embodiment has the advantage of being able to penetrate pockets and corners in the skin for closer elution of NO on the area to be treated.

The device elutes nitric oxide (NO) from said eluting polymer in a therapeutic dose, such as between 0.001 to 5000 ppm, such as 0.01 to 3000 ppm, such as 0.1 to 1000 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58; 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm. The concentration may vary widely depending on where the concentration is measured. If the concentration is measured close to the actual NO eluting polymer the concentration may be as high as thousands of ppm, while the concentration inside the tissue in this case often is considerably lower, such as between 1 to 1000 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these.

Three important factors in controlling and regulating the elution of nitric oxide from a nitric oxide eluting polymer are how quickly a proton donor comes in contact with the nitric oxide releasing polymer, such as a diazoliumdiolate group, the acidity of the environment surrounding the nitric oxide eluting polymer, and the temperature of the environment surrounding the nitric oxide releasing polymer (higher temperature promotes elution of nitric oxide).

In one embodiment of the present invention a nitric oxide eluting polymer, such as L-PEI-NO, is mixed with a carrier polymer to slow down or prolong the elution of nitric oxide. Also, in another embodiment, the nitric oxide eluting polymer may be mixed with more than one carrier polymer, whereby be elution or release may be tailor made to fit specific needs. Such a need may for example be a low elution during a first period of time, when the environment of the nitric oxide eluting polymer is hydrophobic, and a faster elution during a second period of time, when the environment of the nitric oxide eluting polymer has been altered to be more hydrophilic. This may for example be accomplished by using biodegradable polymers, whereby a low elution during a first period of time is obtained, after which, when the hydrophobic polymer has been dissolved, the hydrophilic polymer provides a higher elution of nitric oxide. Thus, a more hydrophobic carrier polymer will give a slower elution of nitric oxide, since the activating proton donor, such as water or body fluid, will penetrate the carrier polymer slower. On the other hand, a hydrophilic polymer acts the opposite way. One example of an hydrophilic polymer is polyethylene oxide, and one example of an hydrophobic polymer is polystyrene. These carrier polymers may be mixed with the nitric oxide eluting polymer and then electrospun to suitable fibers. The skilled person in the art knows which other polymers may be used for similar purposes.

In one embodiment this carrier polymer is substituted by another material with hydrophobic or hydrophilic properties. Therefore, the term "carrier material" in the present context should be interpreted to include carrier polymers and other materials with hydrophilic or hydrophobic properties.

In another embodiment of the present invention the elution of nitric oxide from a nitric oxide eluting polymer, such as L-PEI-NO, is influenced by the presence of protons. This means that a more acidic environment provides a quicker elution of nitric oxide. By activating the nitric oxide eluting polymer, or mixture of nitric oxide eluting polymer and carrier material, with an acidic fluid, such as an ascorbic acid solution, the elution of nitric oxide may be accelerated.

The carrier polymers and carrier materials mentioned in above may affect other characteristics than the regulation of nitric oxide elution. An example of such characteristic is mechanical strength.

In respect of the carrier polymers or carrier materials, the NO-eluting polymer may be integrated in, spun together with, or spun on top of, any of these materials in all of the embodiments of the present invention. This spinning includes electrospinning, air spinning, wet spinning, dry spinning, melt spinning, and gel spinning. In this way, one may manufacture fibers of a polymer mixture, comprising a nitric oxide eluting polymer and a carrier polymer, or a carrier material, with predefined nitric oxide eluting characteristics. These characteristics may be tailor made for different elution profiles in different applications.

The NO-eluting polymers in the devices may be combined with silver, such as hydroactivated silver. The integration of silver in the devices gives the healing process an extra boost. Preferably the silver is releasable from the devices in the form of silver ions. The integration of silver in the device may present several advantages. One example of such an advantage is that the silver may keep the device in itself free from bacteria or viruses, while the nitric oxide eluting polymer elutes the therapeutic dosage of nitric oxide to the target site.

The nitric oxide eluting polymer may comprise a secondary amine, either in the backbone or as a pendant, as described previously. This will make a good nitric oxide eluting polymer. The secondary amine should have a strong negative charge to be easy to load with nitric oxide. If there is a ligand close to the secondary amine, such as on a neighbour atom, such as a carbon atom, to the nitrogen atom, with higher electronegativity than nitrogen (N), it is very difficult to load the polymer with nitric oxide. On the other hand, if there is a electropositive ligand close to the secondary amine, such as on a neighbour atom, such as a carbon atom, to the nitrogen atom, the electronegativity of the amine will increase and thereby increase the possibility to load the nitric oxide elution polymer with nitric oxide.

In an embodiment of the present invention the nitric oxide polymer may be stabilized with a salt. Since the nitric oxide eluting group, such as a diazeniumdiolate group, usually is negative, a positive counter ion, such as a cation, may be used to stabilize the nitric oxide eluting group. This cation may for example be selected from the group comprising any cation from group 1 or group 2 in the periodic table, such as Na⁺, K⁺, Li⁺, Be²⁺, Ca²⁺, Mg ²⁺, Ba²⁺, and/or Sr²⁺. Different salts of the same nitric oxide eluting polymer have different properties. In this way a suitable salt (or cation) may be selected for different purposes. Examples of cationic stabilized polymers are L-PEI-NO-Na, i.e. L-PEI diazeniumdiolate stabilized with sodium, and L-PEI-NO-Ca, i.e. L-PEI diazeniumdiolate stabilized with calcium.

Another embodiment of the present invention comprises mixing the nitric oxide eluting polymer, or a mixture of the nitric oxide eluting polymer and a carrier material, with an absorbent agent. This embodiment provides the advantage of an accelerated elution of nitric oxide since the polymer, or polymer mixture, via the absorbent agent, may take up the activating fluid, such as water or body fluid, much faster. In one example 80 % (w/w) absorbent agent is mixed with the nitric oxide eluting polymer, or mixture of nitric oxide eluting polymer and carrier material, and in another embodiment 10 to 50 % (w/w) absorbent agent is mixed with the nitric oxide eluting polymer, or mixture of nitric oxide eluting polymer and carrier material.

Since the elution of nitric oxide is activated by a proton donor, such as water, it may be an advantage to keep the nitric oxide eluting polymer, or mixture of nitric oxide eluting polymer and carrier material, in contact with said proton donor. If an indication requires an elution of nitric oxide during a prolonged period of time, a system is advantageous, which presents the possibility to keep the proton donor in contact with the nitric oxide eluting polymer, or mixture of nitric oxide eluting polymer and carrier material. Therefore, in still another embodiment of the present invention, the elution of nitric oxide may be regulated by adding an absorbent agent. The absorbent agent absorbs the proton donor, such as water, and keeps the proton donor in close contact with the nitric oxide eluting polymer during prolonged periods of time. Said absorbent agent may be selected from the group comprising polyacrylates, polyethylene oxide, carboxymethylcellulose, and microcrystalline cellulose, cotton, and starch. This absorbent agent may also be used as a filling agent. In this case said filling agent may give the nitric oxide eluting polymer, or mixture of said nitric oxide eluting polymer and a carrier material, a desired texture.

The device may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

It is also within the scope of the present invention to electro spin a NO-eluting polymer together with other suitable polymer/polymers.

Gas stream spinning, air spinning, wet spinning, dry spinning, melt spinning, and gel spinning, of said NO-eluting polymers onto the device is also within the scope of the present invention.

The manufacturing process presents the advantages of large contact surface of the NO-eluting polymer fibres with the area to be treated, effective use of NO-eluting polymer, and a cost effective way of producing the device.

Hereinafter, some potential uses of the present invention are described:
Treating an infection, including onychomycosis and dermatophytosis by means of a device that comprises a nitric oxide (NO) eluting polymer configured for eluting a therapeutic dosage of nitrogen oxide (NO) when used for said treatment, comprising exposing said treatment site of said infection in or on a body to said nitric oxide when said polymer in use elutes nitrogen oxide (NO) by eluting a therapeutic dose of nitric oxide from said nitric oxide eluting polymer to said treatment site.

The use according to the above, wherein said site of said infection is an extremity of a body, and wherein said method comprises applying a condom/sheath, sock, patch/pad, and tape/coating to said extremity for said exposure.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided as a clarifying example.

## Claims

1. A device configured to therapeutically treat dermatomychosis including onychomycosis and/or dermatophytosis,
**characterized in that**
said device comprises a nitric oxide (NO) eluting polymer configured to elute a therapeutic dosage of nitrogen oxide (NO) when used for said treatment, and
wherein said nitric oxide (NO) eluting polymer is integrated with a carrier material, such that said carrier material, in use, regulates and controls the elution of said therapeutic dosage of nitric oxide (NO),
wherein said device is configured to expose a treatment site of said infection, in or on a body, to said nitric oxide when said polymer in use elutes nitrogen oxide (NO), and wherein said elution of nitric oxide (NO) from said device in use substantially is directed towards said target site for said exposure.

2. Device according to claim 1, comprising a first membrane, which is permeable to nitric oxide, on a first side of the device, said first side in use is oriented towards said treatment site, and a second membrane, which has low permeability or substantially no permeability to nitric oxide, on a second side of said device, which in use is oriented away from said treatment site, such that said substantial direction of nitric oxide (NO) from said device in use thereof is provided as the elution of nitric oxide from said device in use is substantially prevented from said second side.

3. Device according to claim 1 or 2, wherein said nitric oxide (NO) eluting polymer comprises diazeniumdiolate groups, S-nitrosylated groups, and O-nitrosylated groups, or any combination these.

4. Device according to claim 1, 2 or 3, wherein said nitric oxide (NO) eluting polymer is L-PEI (linear polyethyleneimine), loaded with nitric oxide (NO) through said diazeniumdiolate groups, S-nitrosylated groups, or O-nitrosylated groups, or any combination these, arranged for release of the nitric oxide (NO) at said target site in or on a body for treatment of or prevention infections, including onychomycosis and dermatophytosis thereby.

5. Device according to claim 1 or 2, wherein said nitric oxide eluting polymer is selected from the group comprising amino cellulose, amino dextrans, chitosan, aminated chitosan, polyethyleneimine, PEI-cellulose, polypropyleneimine, polybutyleneimine, polyurethane, poly(buthanediol spermate), poly(iminocarbonate), polypeptide, Carboxy Methyl Cellulose (CMC), polystyrene, poly(vinyl chloride), and polydimethylsiloxane, or any combinations of these, and these mentioned polymers grafted to an inert backbone, such as a polysaccharide backbone or cellulosic backbone.

6. Device according to claim 1 or 2, has a form selected from the group consisting of a condom/sheath, a sock, a patch/pad, and a tape/coating, adapted to be applied on or at said treatment site of said infection in or on a body for treatment of infections, including onychomycosis and dermatophytosis.

7. Device according to claim 6, wherein said condom/sheath, sock, patch/pad, and tape/coating is manufactured of polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these, and said condom/sheath, sock, patch/pad, or tape/coating, includes said nitric oxide (NO) eluting polymer configured to, in use, elute said nitric oxide (NO) to said treatment site of said infection in or on a body for treatment of said infection.

8. Device according to any of claims 1 to 7, including a proton donor bag, sealed proton donor sponge or proton donor micro capsules, configured for releasing said proton donor therefrom when activated to said device, and wherein said polymer is activateable to elute nitric oxide (NO) upon contact with said proton donor.

9. Device according to claim 1, wherein said device is partly disintegrable when subjected to a proton donor.

10. Device according to claim 8 or 9, wherein said proton donor is selected from the group comprising water, blood, lymph, bile, methanol, ethanol, propanols, buthanols, pentanols, hexanols, phenols, naphtols, polyols, phosphates, succinates, carbonates, acetates, formats, propionates, butyrates, fatty acids, amino acids, or any combinations of these.

11. Device according to claim 10, said proton donor having added a surfactant thereto, said surfactant in use facilitating wettening of the device.

12. Device according to claim 1, wherein said polymer comprises silver, configured for therapeutical treatment of said site of said infection in or on the body.

13. Device according to claim 1 or 2, wherein said polymer is comprised in the device in form of nano-particles or micro-spheres.

14. Device according to claim 13, wherein said nano-particles, or micro-spheres, are integrated in a gel, cream, foam, or hydrogel, or combinations thereof.

15. Device according to claim 13 or 14, wherein said nano-particles, or micro-spheres, are integrated with, preferably encapsulated in, a material, selected from the group comprising polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these.

16. Device according to claim 1 or 2, wherein said carrier material is selected from the group comprising polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these.

17. Device according to claim 1 or 2, comprising an absorbent agent, configured to absorb a proton donor, and to thereby keep said proton donor in close contact with the nitric oxide eluting polymer during prolonged periods of time.

18. Device according to claim 17, wherein said absorbent agent is selected from the group comprising polyacrylates, polyethylene oxide, carboxymethylcellulose, and microcrystalline cellulose, cotton, and starch.

19. Device according to claim 1 or 2, wherein said nitric oxide eluting polymer is stabilized by a cation.

20. Device according to claim 19, wherein said cation is selected from the group comprising Na⁺, K⁺, Li⁺, Be²⁺, Ca²⁺, Mg²⁺, Ba²⁺, and Sr²⁺, or any combinations thereof.

21. Device according to claim 1 or 2, wherein said nitric oxide eluting polymer comprises a secondary amine, either in the backbone or as a pendant.

22. Device according to claim 21, wherein a positive ligand is located close to said secondary amine.

23. Device according to claim 21, wherein said electropositive ligand is located on a neighbour carbon atom to the nitrogen atom in said secondary amine in the backbone.

24. Device according to any preceding claim, wherein said device is configured to therapeutically treat onychomycosis or dermatophytosis.

25. A manufacturing process for a device configured to therapeutically treat dermatomychosis, including onychomycosis and dermatophytosis, according to claim 1, comprising:
selecting a nitric oxide (NO) eluting polymer configured to elute a therapeutic dosage of nitric oxide (NO) when used for said therapeutic treatment of infections,
selecting a carrier material, which carrier material is configured to regulate and control the elution of said therapeutic dosage of nitric oxide (NO),
incorporating the NO-eluting polymer with said carrier material into an nitric oxide (NO) eluting material, such that said carrier material, in use of said device, regulates and controls the elution of said therapeutic dosage of nitric oxide (NO), and
deploying said nitric oxide eluting material into a suitable form, or as a coating onto a carrier, to form at least a part of said device, such that said device is configured to expose a therapeutic target site to said nitric oxide when said NO-eluting polymer in use elutes nitric oxide (NO), and
applying a material that has low permeability or substantially no permeability to nitric oxide (NO) on a side of device that is intended to be oriented away from said therapeutic target site, such that elution of nitric oxide in use substantially is directed towards said therapeutic target site.

26. The manufacturing process according to claim 25,
wherein said deploying comprises electro spinning, air spinning, gas spinning, wet spinning, dry spinning, melt spinning, or gel spinning of NO-eluting polymer.

27. The manufacturing process according to claim 25 or 26, wherein said selecting said nitric oxide (NO) eluting polymer comprises selecting a plurality of nitric oxide (NO) eluting polymeric particles, preferably nano fibres, nano particles or micro spheres.

28. The manufacturing process according to claim 25 or 26, wherein said incorporating said NO-eluting polymer with said carrier material comprises integrating said NO-eluting polymer in said carrier material, spinning said NO-eluting polymer together with said carrier material, or spinning said NO-eluting polymer on top of said carrier material, in order to predefine nitric oxide eluting characteristics of said device..

29. The manufacturing process according to claim 25, further comprising integrating silver in said device.

30. The manufacturing process according to claim 25, further comprising microencapsulating a proton donor in micro capsules, and
applying the micro capsules to said nitric oxide (NO) eluting material.

31. The manufacturing process according to claim 30, wherein said applying comprises pattern gluing, or spinning the NO eluting material onto said micro capsules.

32. The manufacturing process according to claim 31, comprising forming the micro capsules into a first film, tape, or sheath,
forming a second film, tape, or sheath of said NO eluting material, and
gluing the first film, tape, or sheath of micro capsules to said second film, tape, or sheath of said NO eluting material.

33. The manufacturing process according to claim 32, wherein said gluing comprises patterned gluing, such that a pattern is obtained including glue free spaces.

34. The manufacturing process according to claim 30, comprising forming the micro capsules into a first film, tape, or sheath, and directly spinning the NO eluting material onto the film, tape, or sheath of micro capsules, containing the proton donor.

35. The manufacturing process according to claim 30, comprising providing an activation indicator configured to indicate when the micro capsules are broken such that the NO eluting material is subjected to said proton donor to elute NO therefrom, regulated and/or controlled by said carrier material.

36. The manufacturing process according to claim 35, wherein said providing an activation indicator comprises providing a coloring agent inside the micro capsules.

37. The manufacturing process according to claim 35, wherein said providing an activation indicator comprises selecting a material for the micro capsules, or choosing a wall thickness of said micro capsules, that creates a sound when the micro capsules break.

38. The manufacturing process according to claim 35, wherein said providing an activation indicator comprises admixing a scent material into the micro capsules.

39. The manufacturing process according to claim 35, wherein said providing an activation indicator comprises providing a substance that changes color when it comes in contact with the proton donor.

40. Use of a nitric oxide (NO) eluting polymer for the manufacture of a device according to any of claims 1-24 for the treatment of dermatomychosis, including onychomycosis and dermatophytosis
wherein
nitric oxide is loaded to said device so said device elutes nitric oxide (NO) from said eluting polymer in a therapeutic dose when used at a site of dermatomychosis on a body towards said site.

41. Use according to claim 40, wherein said therapeutic dose is between 0.001 to 5000 ppm, such as 0.01 to 3000 ppm, such as 0.1 to 1000 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

42. A nitric oxide (NO) eluting polymer for use in the treatment of dermatomychosis, including onychomycosis and dermatophytosis, wherein
nitric oxide is loaded to a device so said device elutes nitric oxide (NO) from said eluting polymer in a therapeutic dose when used at a site of dermatomychosis on a body towards said site.

43. The nitric oxide (NO) polymer according to claim 42, wherein said therapeutic dose is between 0.001 to 5000 ppm, such as 0.01 to 3000 ppm, such as 0.1 to 1000 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

## Patentansprüche

1. Vorrichtung, die dafür ausgelegt ist, Dermatomykose einschließlich von Nagelmykose und/oder Dermatophytie therapeutisch zu behandeln,
**dadurch gekennzeichnet, dass**
die Vorrichtung ein Stickoxid (NO) eluierendes Polymer aufweist, das dafür ausgelegt ist, eine therapeutische Stickoxid (NO)-Dosis zu eluieren, wenn es für die Behandlung gebraucht wird, und
wobei das Stickoxid (NO) eluierende Polymer mit einem Trägermaterial vereinigt ist, so dass das Trägermaterial im Gebrauch die Elution der therapeutischen Stickoxid (NO)-Dosis reguliert und steuert,
wobei die Vorrichtung dafür ausgelegt ist, das Stickoxid auf eine zu behandelnde infizierte Stelle im oder am Körper einwirken zu lassen, wenn das Polymer im Gebrauch Stickoxid (NO) eluiert, und wobei die Elution von Stickoxid (NO) aus der Vorrichtung im Gebrauch im Wesentlichen auf die Zielstelle gerichtet ist, um auf diese einzuwirken.

2. Vorrichtung nach Anspruch 1, mit einer ersten Membran, die für Stickoxid durchlässig ist, auf einer ersten Seite der Vorrichtung, wobei die erste Seite im Gebrauch der Behandlungsstelle zugewandt ist, sowie einer zweiten Membran, die eine geringe Durchlässigkeit oder im Wesentlichen keine Durchlässigkeit für Stickoxid hat, auf einer zweiten Seite der Vorrichtung, die im Gebrauch von der Behandlungsstelle abgewandt ist, so dass die wesentliche Richtung des Stickoxids (NO) aus der Vorrichtung bei deren Gebrauch vorgegeben ist, da die Elution von Stickoxid aus der Vorrichtung von der zweiten Seite im Gebrauch im Wesentlichen verhindert wird.

3. Vorrichtung nach Anspruch 1 oder 2, wobei das Stickoxid (NO) eluierende Polymer Diazeniumdiolat-Gruppen, S-nitrosylierte Gruppen und O-nitrosylierte Gruppen oder eine beliebige Kombination davon aufweist.

4. Vorrichtung nach Anspruch 1, 2 oder 3, wobei das Stickoxid (NO) eluierende Polymer L-PEI (lineares Polyethlyenimin) ist, das durch die Diazeniumdiolat-Gruppen, S-nitrosylierten Gruppen oder O-nitrosylierten Gruppen oder eine beliebige Kombination von diesen mit Stickoxid (NO) beladen wird und das für die Freisetzung des Stickoxids (NO) an der Zielstelle in oder an einem Körper ausgelegt ist, um Infektionen, einschließlich von Nagelmykose und Dermatophytie, zu behandeln oder zu verhüten.

5. Vorrichtung nach Anspruch 1 oder 2, wobei das Stickoxid eluierende Polymer ausgewählt ist aus der Gruppe, die Aminocellulose, Aminodextrane, Chitosan, aminiertes Chitosan, Polyethylenimin, PEI-Cellulose, Polypropylenimin, Polybutylenimin, Polyurethan, Poly(butandiolspermat), Poly(iminocarbonat), Polypeptid, Carboxymethylcellulose (CMC), Polystyrol, Poly(vinylchlorid) und Polydimethylsiloxan oder irgendeine Kombination von diesen einschließt, und diese genannten Polymere auf eine inerte Hauptkette, wie eine Polysaccharidhauptkette oder eine cellulosische Hauptkette, gepfropft sind.

6. Vorrichtung nach einem der Ansprüche 1 oder 2, die eine Gestalt aufweist, die ausgewählt ist aus der Gruppe bestehend aus einem Kondom / einer Hülle, einer Socke, einem Pflaster / einem Pad und einem Streifen / einer Auflage, und die dafür ausgelegt ist, auf oder an die zu behandelnde infizierte Stelle in oder an einem Körper gelegt zu werden, um Infektionen, einschließlich von Nagelmykose und Dermatophytie, zu behandeln.

7. Vorrichtung nach Anspruch 6, wobei das Kondom / die Hülle, die Socke, das Pflaster / das Pad und der Streifen / die Auflage aus Polyethylen, Polypropylen, Polyacrylonitril, Polyurethan, Polyvinylacetaten, Polymilchsäuren, Stärke, Cellulose, Polyhydroxyalkanoaten, Polyestern, Polycaprolacton, Polyvinylalkohol, Polystyrol, Polyethern, Polycarbonaten, Polyamiden, Polyolefinen, Poly(acrylsäure), Carboxymethylcellulose (CMC), Polymeren auf Proteinbasis, Gelatine, biologisch abbaubaren Polymeren, Baumwolle und Latex oder einer beliebigen Kombination davon gefertigt sein kann, und das Kondom / die Hülle, die Socke, das Pflaster / das Pad und der Streifen / die Auflage das Stickoxid (NO) eluierende Polymer enthält, das dafür ausgelegt ist, im Gebrauch das Stickoxid (NO) an die zu behandelnde infizierte Stelle in oder an einem Körper zu eluieren, um die Infektion zu behandeln.

8. Vorrichtung nach einem der Ansprüche 1 bis 7, einen Protonendonorbeutel, einen versiegelten Protonendonorschwamm oder Protonendonormikrokapseln auf weisend, der bzw. die dafür ausgelegt ist bzw. sind, den Protonendonor an die Vorrichtung abzugeben, wenn er bzw. sie aktiviert wurde(n), und wobei das Polymer aktivierbar ist, um bei Kontakt mit dem Protonendonor Stickoxid (NO) zu eluieren.

9. Vorrichtung nach Anspruch 1, wobei die Vorrichtung zum Teil zerfallen kann, wenn sie einem Protonendonor ausgesetzt wird.

10. Vorrichtung nach Anspruch 8 oder 9, wobei der Protonendonor ausgewählt ist aus der Gruppe, die Wasser, Blut, Lymphe, Galle, Methanol, Ethanol, Propanole, Butanole, Pentanole, Hexanole, Phenole, Naphthole, Polyole, Phosphate, Succinate, Carbonate, Acetate, Formiate, Propionate, Butyrate, Fettsäuren, Aminosäuren oder irgendwelchen Kombinationen davon einschließt.

11. Vorrichtung nach Anspruch 10, wobei dem Protonendonor ein Tensid hinzugefügt wurde, wobei das Tensid im Gebrauch die Benetzung der Vorrichtung erleichtert.

12. Vorrichtung nach Anspruch 1, wobei das Polymer Silber aufweist und für die therapeutische Behandlung der infizierten Stelle im oder am Körper ausgelegt ist.

13. Vorrichtung nach Anspruch 1 oder 2, wobei das Polymer in der Vorrichtung in Form von Nanopartikeln oder Mikrokügelchen enthalten ist.

14. Vorrichtung nach Anspruch 13, wobei die Nanopartikel oder Mikrokügelchen in ein Gel, eine Creme, einen Schaum oder ein Hydrogel oder eine Kombination davon integriert sind.

15. Vorrichtung nach Anspruch 13 oder 14, wobei die Nanopartikel oder Mikrokügelchen in ein Material integriert und vorzugsweise eingekapselt sind, das ausgewählt ist aus der Gruppe, die Polyethylen, Polypropylen, Polyacrylonitril, Polyurethan, Polyvinylacetate, Polymilchsäuren, Stärke, Cellulose, Polyhydroxyalkanoate, Polyester, Polycaprolacton, Polyvinylalkohol, Polystyrol, Polyether, Polycarbonate, Polyamide, Polyolefine, Poly(acrylsäure), Carboxymethylcellulose (CMC), Polymere auf Proteinbasis, Gelatine, biologisch abbaubare Polymere, Baumwolle und Latex oder eine beliebige Kombination davon einschließt.

16. Vorrichtung nach Anspruch 1 oder 2, wobei das Trägermaterial ausgewählt ist aus der Gruppe, die Polyethylen, Polypropylen, Polyacrylonitril, Polyurethan, Polyvinylacetate, Polymilchsäuren, Stärke, Cellulose, Polyhydroxyalkanoate, Polyester, Polycaprolacton, Polyvinylalkohol, Polystyrol, Polyether, Polycarbonate, Polyamide, Polyolefine, Poly(acrylsäure), Carboxymethylcellulose (CMC), Polymere auf Proteinbasis, Gelatine, biologisch abbaubare Polymere, Baumwolle und Latex oder eine beliebige Kombination davon einschließt.

17. Vorrichtung nach Anspruch 1 oder 2, ein Absorptionsmittel aufweisend, das so ausgelegt ist, dass es einen Protonendonor absorbiert und **dadurch** den Protonendonor über längere Zeiträume in engem Kontakt mit dem Stickoxid eluierenden Polymer hält.

18. Vorrichtung nach Anspruch 17, wobei das Absorptionsmittel ausgewählt ist aus der Gruppe, die Polyacrylate, Polyethylenoxid, Carboxymethylcellulose und mikrokristalline Cellulose, Baumwolle und Stärke einschließt.

19. Vorrichtung nach Anspruch 1 oder 2, wobei das Stickoxid eluierende Polymer durch ein Kation stabilisiert wird.

20. Vorrichtung nach Anspruch 19, wobei das Kation ausgewählt ist aus der Gruppe, die Na⁺, K⁺, Li⁺, Be²⁺, Ca²⁺, Mg² , Ba²⁺ und Sr²⁺ oder eine beliebige Kombination davon einschließt.

21. Vorrichtung nach Anspruch 1 oder 2, wobei das Stickoxid eluierende Polymer entweder in der Hauptkette oder seitenständig ein sekundäres Amin aufweist.

22. Vorrichtung nach Anspruch 21, wobei ein positiver Ligand sich nahe am sekundären Amin befindet.

23. Vorrichtung nach Anspruch 21, wobei der elektropositive Ligand sich auf einem dem Stickstoffatom in dem sekundären Amin benachbarten Kohlenstoffatom in der Hauptkette befindet.

24. Vorrichtung nach einem der vorangehenden Ansprüche, wobei die Vorrichtung für eine therapeutische Behandlung von Nagelmykose oder Dermatophytie ausgelegt ist.

25. Herstellungsverfahren für eine Vorrichtung gemäß Anspruch 1, die für eine therapeutische Behandlung von Dermatomykose, einschließlich von Nagelmykose und Dermatophytie, ausgelegt ist, umfassend:
Auswählen eines Stickoxid (NO) eluierenden Polymers, das dafür ausgelegt ist, eine therapeutische Stickoxid (NO)-Dosis zu eluieren, wenn es für die therapeutische Behandlung von Infektionen gebraucht wird,
Auswählen eines Trägermaterials, wobei das Trägermaterial dafür ausgelegt ist, die Elution der therapeutischen Stickoxid (NO)-Dosis zu regulieren und zu steuern,
Vereinigen des NO-eluierenden Polymers mit dem Trägermaterial zu einem Stickoxid (NO) eluierenden Material, so dass das Trägermaterial, wenn die Vorrichtung im Gebrauch ist, die Elution der therapeutischen Stickoxid (NO)-Dosis reguliert und steuert, und
Einbringen des Stickoxid eluierenden Materials in eine geeignete Form oder als Beschichtung auf einen Träger, um mindestens einen Teil der Vorrichtung zu bilden, so dass die Vorrichtung dafür ausgelegt wird, Stickoxid auf eine therapeutische Zielstelle einwirken zu lassen, wenn das NO-eluierende Polymer im Gebrauch Stickoxid (NO) eluiert, und
Aufbringen eines Materials, das eine geringe Durchlässigkeit oder im Wesentlichen keine Durchlässigkeit für Stickoxid (NO) hat, auf eine Seite der Vorrichtung, die dafür bestimmt ist, von der therapeutischen Zielstelle abgewendet zu werden, so dass eine Elution von Stickoxid im Gebrauch im Wesentlichen auf die therapeutische Zielstelle gerichtet wird.

26. Herstellungsverfahren nach Anspruch 25, wobei das Einbringen ein Elektrospinnen, Luftspinnen, Gasspinnen, Nassspinnen, Trockenspinnen, Schmelzspinnen oder Gelspinnen von NO-eluierendem Polymer umfasst.

27. Herstellungsverfahren nach Anspruch 25 oder 26, wobei das Auswählen des Stickoxid (NO) eluierenden Polymers das Auswählen einer Vielzahl von Stickoxid (NO) eluierenden Polymerteilchen, vorzugsweise Nanofasern, Nanoteilchen oder Mikrokügelchen, umfasst.

28. Herstellungsverfahren nach Anspruch 25 oder 26, wobei das Vereinigen des NO eluierenden Polymers mit dem Trägermaterial das Integrieren des NO-eluierenden Polymers in das Trägermaterial, das Verspinnen des NO-eluierenden Polymers zusammen mit dem Trägermaterial oder das Spinnen des NO-eluierenden Polymers über dem Trägermaterial umfasst, um Stickoxid-eluierende Eigenschaften der Vorrichtung vorab zu definieren.

29. Herstellungsverfahren nach Anspruch 25, ferner das Integrieren von Silber in die Vorrichtung umfassend.

30. Herstellungsverfahren nach Anspruch 25, ferner die Mikroverkapselung eines Protonendonors in Mikrokapseln und
das Aufbringen der Mikrokapseln auf das Stickoxid (NO) eluierende Material umfassend.

31. Herstellungsverfahren nach Anspruch 30, wobei das Aufbringen ein gemustertes Aufleimen oder Spinnen des NO-eluierenden Materials auf die Mikrokapseln umfasst.

32. Herstellungsverfahren nach Anspruch 31, das Ausbilden der Mikrokapseln als eine erste Folie, einen erstes Streifen oder eine erste Hülle,
das Ausbilden einer zweiten Folie, eines zweiten Streifens oder einer zweiten Hülle aus dem NO-eluierenden Material und
das Leimen der ersten Folie, des ersten Streifens oder der ersten Hülle aus Mikrokapseln auf die zweite Folie, den zweiten Streifen oder die zweite Hülle aus dem NO-eluierenden Material umfassend.

33. Herstellungsverfahren nach Anspruch 32, wobei das Leimen ein gemustertes Leimen umfasst, so dass ein Muster erhalten wird, das leimfreie Stellen aufweist.

34. Herstellungsverfahren gemäß Anspruch 30, das Ausbilden der Mikrokapseln als eine erste Folie, einen ersten Streifen oder eine erste Hülle und das direkte Spinnen des NO-eluierenden Materials auf die Folie, den Streifen oder die Hülle aus Mikrokapseln, welche den Protonendonor enthalten, umfassend.

35. Herstellungsverfahren gemäß Anspruch 30, das Bereitstellen eines Aktivierungsindikators umfassend, der dafür ausgelegt ist, anzuzeigen, wenn die Mikrokapseln zerbrochen werden, so dass das NO-eluierende Material dem Protonendonor ausgesetzt wird, um, reguliert und/oder gesteuert vom Trägermaterial, NO daraus zu eluieren

36. Herstellungsverfahren nach Anspruch 35, wobei das Bereitstellen eines Aktivierungsindikators das Bereitstellen eines Färbemittels innerhalb der Mikrokapseln umfasst.

37. Herstellungsverfahren nach Anspruch 35, wobei das Bereitstellen eines Aktivierungsindikators das Auswählen eines Materials für die Mikrokapseln oder das Auswählen einer Wanddicke der Mikrokapseln umfasst, wodurch ein Ton erzeugt wird, wenn die Mikrokapseln zerbrechen.

38. Herstellungsverfahren nach Anspruch 35, wobei das Bereitstellen eines Aktivierungsindikators das Beimischen eines Geruchsstoffes in die Mikrokapseln umfasst.

39. Herstellungsverfahren nach Anspruch 35, wobei das Bereitstellen eines Aktivierungsindikators das Bereitstellen einer Substanz, die ihre Farbe ändert, wenn sie mit dem Protonendonor in Kontakt kommt, umfasst.

40. Verwendung eins Stickoxid (NO) eluierenden Polymers für die Herstellung einer Vorrichtung nach einem der Ansprüche 1 - 24 für die Behandlung von Dermatomykose, einschließlich von Nagelmykose und Dermatophytie, wobei
Stickoxid in die Vorrichtung geladen wird, so dass die Vorrichtung, wenn sie an einer Dermatomykosestelle an einem Körper eingesetzt wird, Stickoxid (NO) aus dem eluierenden Polymer in einer therapeutischen Dosis in Richtung auf die Stelle eluiert.

41. Verwendung nach Anspruch 40, wobei die therapeutische Dosis zwischen 0,001 und 5000 ppm liegt, wie 0,01 bis 3000 ppm, wie 0,1 bis 1000 ppm, wie 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 oder 100 ppm.

42. Ein Stickoxid (NO) eluierendes Polymer für die Behandlung von Dermatomykose, einschließlich von Nagelmykose und Dermatophytie, wobei Stickoxid in ein Vorrichtung geladen wird, so dass die Vorrichtung, wenn sie an einer Dermatomykosestelle an einem Körper eingesetzt wird, Stickoxid (NO) aus dem eluierenden Polymer in einer therapeutischen Dosis in Richtung auf die Stelle eluiert.

43. Verwendung des Stickoxid (NO) Polymers nach Anspruch 42, wobei die therapeutische Dosis zwischen 0,001 und 5000 ppm liegt, wie 0,01 bis 3000 ppm, wie 0,1 bis 1000 ppm, wie 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 oder 100 ppm.

## Revendications

1. Dispositif agencé pour le traitement thérapeutique de la dermatomycose, y compris l'onychomycose et/ou la dermatophytose,
**caractérisé en ce que**
ledit dispositif comprend un polymère éluant l'oxyde nitrique (NO) agencé de façon à éluer une dose thérapeutique d'oxyde nitrique (NO) lorsqu'il est utilisé pour ledit traitement, et
dans lequel ledit polymère éluant l'oxyde nitrique (NO) est intégré à un matériau porteur, de sorte que ledit matériau porteur, en utilisation, régule et contrôle l'élution de ladite dose thérapeutique d'oxyde nitrique (NO),
dans lequel ledit dispositif est agencé de façon à exposer un site de traitement de ladite infection, dans ou sur un corps, audit oxyde nitrique lorsque le polymère en utilisation élue de l'oxyde nitrique (NO), et dans lequel ladite élution d'oxyde nitrique (NO) à partir dudit dispositif en utilisation est principalement dirigée vers ledit site cible pour ladite exposition.

2. Dispositif selon la revendication 1, comprenant une première membrane, qui est perméable à l'oxyde nitrique, sur un premier côté du dispositif, ledit premier côté en utilisation étant orienté vers ledit site de traitement, et une deuxième membrane, qui présente peu ou pas de perméabilité à l'oxyde nitrique, sur un deuxième côté dudit dispositif, qui en utilisation est orienté dans le sens opposé audit site de traitement, de sorte que la direction de l'oxyde nitrique (NO) à partir dudit dispositif en utilisation est sensiblement assurée, alors que l'élution de l'oxyde nitrique à partir dudit dispositif en utilisation est sensiblement interdite sur le deuxième côté.

3. Dispositif selon la revendication 1 ou 2, dans lequel ledit polymère éluant l'oxyde nitrique (NO) comprend des groupements diazénium diolates, des groupements S-nitrosylatés, et des groupements O-nitrosylatés, ou n'importe quelle combinaison de ces derniers.

4. Dispositif selon la revendication 1, 2 ou 3, dans lequel ledit polymère éluant l'oxyde nitrique (NO) est de type PEI-L (polyéthylène-imine linéaire), chargé avec de l'oxyde nitrique (NO) par l'intermédiaire desdits groupements diazeniumdiolates, groupements S-nitrosylés, ou groupements O-nitrosylés, ou n'importe quelle combinaison de ces derniers, agencés pour libérer de l'oxyde nitrique (NO) au niveau dudit site de traitement dans ou sur le corps pour le traitement ou la prévention d'infections, y compris l'onychomycose et la dermatophytose.

5. Dispositif selon la revendication 1 ou 2, dans lequel ledit polymère éluant l'oxyde nitrique est choisi dans le groupe comprenant de l'amino-cellulose, des aminodextranes, du chitosane, du chitosane aminé, du polyéthylène-imine, de la PEI-cellulose, du polypropylèneimine, du polybutylène-imine, du polyuréthane, du poly(butanediol spermate), du poly(iminocarbonate), du polypeptide, de la Carboxy-Méthyle Cellulose (CMC), du polystyrène, du polychlorure de vinyle, et du polydimethylsiloxane, ou n'importe quelles combinaisons de ces derniers, ces polymères étant greffés sur un squelette inerte, par exemple un squelette polysaccharide ou un squelette cellulosique.

6. Dispositif selon la revendication 1 ou 2, a une forme choisie dans le groupe comprenant un préservatif/étui, une chaussette, un patch/tampon, et un ruban/revêtement, propre à être appliqué sur ou au niveau dudit site de traitement de ladite infection dans ou sur un corps pour le traitement des infections, y compris l'onychomycose et la dermatophytose.

7. Dispositif selon la revendication 6, dans lequel ledit préservatif/étui, chaussette, patch/tampon, et ruban/revêtement est fabriqué en polyéthylène, polypropylène, polyacrylonitrile, polyuréthane, polyvinylacétates, acides polylactiques, amidon, cellulose, polyhydroxyalcanoates, polyesters, polycaprolactone, alcool de polyvinyle, polystyrène, polyéthers, polycarbonates, polyamides, polyoléfines, poly(acide acrylique), Carboxy-Méthyle Cellulose (CMC), polymères à base de protéines, gélatine, polymères biodégradables, coton, et latex, ou n'importe quelles combinaisons de ces derniers, et ledit préservatif/étui, chaussette, patch/tampon, ou ruban/revêtement, comprend ledit polymère éluant l'oxyde nitrique (NO) agencé de façon à, en utilisation, éluer ledit oxyde nitrique (NO) vers ledit site de traitement de ladite infection dans ou sur un corps pour le traitement de ladite infection.

8. Dispositif selon l'une quelconque des revendications 1 à 7, comprenant une poche à donneurs de protons, une éponge à donneurs de protons scellée ou des microcapsules à donneurs de protons, agencées pour en libérer ledit donneur de protons, une fois activées audit dispositif, et dans lequel ledit polymère peut être activé pour éluer de l'oxyde nitrique (NO) au contact avec ledit donneur de protons.

9. Dispositif selon la revendication 1, dans lequel ledit dispositif est partiellement désintégrable lorsqu'il est soumis à un donneur de protons.

10. Dispositif selon la revendication 8 ou 9, dans lequel ledit donneur de protons est choisi dans le groupe comprenant de l'eau, du sang, de la lymphe, de la bile, du méthanol, de l'éthanol, des propanols, des butanols, des pentanols, des hexanols, des phénols, des naphtols, des polyols, des phosphates, des succinates, des carbonates, des acétates, des formiates, des propionates, des butyrates, des acides gras, des acides aminés, ou n'importe quelles combinaisons de ces derniers.

11. Dispositif selon la revendication 10, un surfactant ayant été ajouté audit donneur de protons, ledit surfactant en utilisation permettant de mouiller le dispositif.

12. Dispositif selon la revendication 1, dans lequel ledit polymère comprend de l'argent, agencé pour le traitement thérapeutique dudit site de ladite infection dans ou sur le corps.

13. Dispositif selon la revendication 1 ou 2, dans lequel ledit polymère est compris dans le dispositif sous la forme de nanoparticules ou micro-sphères.

14. Dispositif selon la revendication 13, dans lequel lesdites nanoparticules, ou micro-sphères, sont intégrées dans un gel, une crème, une mousse ou un hydrogel, ou des combinaisons de ces derniers.

15. Dispositif selon la revendication 13 ou 14, dans lequel lesdites nanoparticules, ou micro-sphères, sont intégrées à, de préférence encapsulées dans, un matériau, choisi dans le groupe comprenant du polyéthylène, du polypropylène, du polyacrylonitrile, du polyuréthane, des polyvinylacétates, des acides polylactiques, de l'amidon, de la cellulose, des polyhydroxyalcanoates, des polyesters, du polycaprolactone, de l'alcool de polyvinyle, du polystyrène, des polyéthers, des polycarbonates, des polyamides, des polyoléfines, du poly(acide acrylique), de la Carboxy-Méthyle Cellulose (CMC), des polymères à base de protéines, de la gélatine, des polymères biodégradables, du coton, et du latex, ou n'importe quelles combinaisons de ces derniers.

16. Dispositif selon la revendication 1 ou 2, dans lequel ledit matériau porteur est choisi dans le groupe comprenant du polyéthylène, du polypropylène, du polyacrylonitrile, du polyuréthane, des polyvinylacétates, des acides polylactiques, de l'amidon, de la cellulose, des polyhydroxyalcanoates, des polyesters, du polycaprolactone, de l'alcool de polyvinyle, du polystyrène, des polyéthers, des polycarbonates, des polyamides, des polyoléfines, du poly(acide acrylique), de la Carboxy-Méthyle Cellulose (CMC), des polymères à base de protéines, de la gélatine, des polymères biodégradables, du coton, et du latex, ou n'importe quelles combinaisons de ces derniers.

17. Dispositif selon la revendication 1 ou 2, comprenant un agent absorbant, agencé de façon à absorber un donneur de protons, et garder ainsi ledit donneur de protons en contact étroit avec le polymère éluant l'oxyde nitrique pendant des durées prolongées.

18. Dispositif selon la revendication 17, dans lequel ledit agent absorbant est choisi dans le groupe comprenant des polyacrylates, de l'oxyde de polyéthylène, du carboxyméthylcellulose, et de la cellulose microcristalline, du coton, et de l'amidon.

19. Dispositif selon la revendication 1 ou 2, dans lequel ledit polymère éluant l'oxyde nitrique est stabilisé par un cation.

20. Dispositif selon la revendication 19, dans lequel ledit cation est choisi dans le groupe comprenant Na⁺, K⁺, Li⁺, Be²⁺ , Ca²⁺, Mg²⁺, Ba²⁺, et Sr²⁺, ou n'importe quelles combinaisons de ces derniers.

21. Dispositif selon la revendication 1 ou 2, dans lequel ledit polymère éluant l'oxyde nitrique comprend une amine secondaire, située dans le squelette ou en pendant.

22. Dispositif selon la revendication 21, dans lequel un ligand positif est situé à proximité de ladite amine secondaire.

23. Dispositif selon la revendication 21, dans lequel ledit ligand électropositif est situé sur un atome de carbone adjacent à l'atome d'azote dans ladite amine secondaire dans le squelette.

24. Dispositif selon l'une quelconque des revendications précédentes, dans lequel ledit dispositif est agencé pour le traitement thérapeutique de l'onychomycose ou de la dermatophytose.

25. Un procédé de fabrication pour un dispositif agencé pour le traitement thérapeutique de la dermatomycose, y compris l'onychomycose et la dermatophytose, selon la revendication 1, comprenant :
la sélection d'un polymère éluant l'oxyde nitrique (NO) agencé de façon à éluer une dose thérapeutique d'oxyde nitrique (NO) lorsqu'il est utilisé pour ledit traitement thérapeutique d'infections,
la sélection d'un matériau porteur, lequel matériau porteur étant agencé de façon à réguler et contrôler l'élution de ladite dose thérapeutique d'oxyde nitrique (NO) ,
l'incorporation du polymère éluant le NO avec ledit matériau porteur dans un matériau éluant l'oxyde nitrique (NO), de sorte que ledit matériau porteur, lors de l'utilisation dudit dispositif, régule et contrôle l'élution de ladite dose thérapeutique d'oxyde nitrique (NO), et
le déploiement dudit matériau éluant l'oxyde nitrique dans une forme appropriée, ou dans une couche sur le porteur, de façon à former au moins une partie dudit dispositif, de sorte que ledit dispositif soit agencé pour exposer un site cible thérapeutique audit oxyde nitrique lorsque ledit polymère éluant le NO en utilisation élue l'oxyde nitrique (NO), et
l'application d'un matériau présentant peu ou pas de perméabilité à l'oxyde nitrique (NO) sur un côté du dispositif qui sera orienté vers le côté opposé dudit site cible thérapeutique, de sorte que l'élution de l'oxyde nitrique en utilisation soit sensiblement orientée vers le site cible thérapeutique.

26. Le procédé de fabrication selon la revendication 25, dans lequel ledit déploiement comprend le filage électrique, le filage air, le filage gaz, le filage humide, le filage à sec, le filage par fusion, ou le filage gel du polymère éluant le NO.

27. Le procédé de fabrication selon la revendication 25 ou 26, dans lequel ladite sélection dudit polymère éluant l'oxyde nitrique (NO) comprend la sélection d'une pluralité de particules polymériques éluant l'oxyde nitrique (NO), de préférence des nanofibres, des nanoparticules ou des micro-sphères.

28. Le procédé de fabrication selon la revendication 25 ou 26, dans lequel ladite incorporation dudit polymère éluant le NO avec ledit matériau porteur comprend l'intégration dudit polymère éluant le NO dans ledit matériau porteur, le filage dudit polymère éluant le NO avec ledit matériau porteur, ou le filage dudit polymère éluant le NO sur le dessus dudit matériau porteur, de façon à prédéfinir les caractéristiques d'élution d'oxyde nitrique dudit dispositif.

29. Le procédé de fabrication selon la revendication 25, comprenant en outre l'intégration d'argent dans ledit dispositif.

30. Le procédé de fabrication selon la revendication 25, comprenant en outre la microencapsulation d'un donneur de protons dans des microcapsules, et
l'application des microcapsules sur ledit matériau éluant l'oxyde nitrique (NO) .

31. Le procédé de fabrication selon la revendication 30, dans lequel ladite application comprend l'encollage à façon ou le filage du matériau éluant le NO sur lesdites microcapsules.

32. Le procédé de fabrication selon la revendication 31, comprenant la formation des microcapsules dans une première pellicule, un premier ruban ou un premier étui,
la formation d'une deuxième pellicule, d'un deuxième ruban ou d'un deuxième étui dudit matériau éluant le NO, et
l'encollage de la première pellicule, le premier ruban ou le premier étui de microcapsules sur la deuxième pellicule, le deuxième ruban ou le deuxième étui dudit matériau éluant le NO.

33. Le procédé de fabrication selon la revendication 32, dans lequel ledit encollage comprend un encollage à façon, de sorte qu'un motif soit obtenu comportant des espaces sans colle.

34. Le procédé de fabrication selon la revendication 30, comprenant la formation de microcapsules dans une première pellicule, un premier ruban ou un premier étui, et le filage du matériau éluant le NO directement sur la pellicule, le ruban ou l'étui de microcapsules, contenant le donneur de protons.

35. Le procédé de fabrication selon la revendication 30, comprenant la fourniture d'un indicateur d'activation agencé de façon à indiquer le moment où les microcapsules se cassent de sorte que le matériau éluant le NO soit soumis audit donneur de protons pour en éluer le NO, régulé et/ou contrôlé par ledit matériau porteur.

36. Le procédé de fabrication selon la revendication 35, dans lequel ladite fourniture d'un indicateur d'activation comprend la fourniture d'un agent colorant dans les microcapsules.

37. Le procédé de fabrication selon la revendication 35, dans lequel ladite fourniture d'un indicateur d'activation comprend la sélection d'un matériau pour les microcapsules, ou la sélection d'une épaisseur de paroi desdites microcapsules, qui produise un son lorsque les microcapsules se cassent.

38. Le procédé de fabrication selon la revendication 35, dans lequel ladite fourniture d'un indicateur d'activation comprend l'addition d'un matériau odorant dans les microcapsules.

39. Le procédé de fabrication selon la revendication 35, dans lequel ladite fourniture d'un indicateur d'activation comprend la fourniture d'une substance qui change de couleur lorsqu'il rentre en contact avec le donneur de protons.

40. L'utilisation d'un polymère éluant l'oxyde nitrique (NO) pour la fabrication d'un dispositif selon l'une quelconque des revendications 1 à 24 pour le traitement de la dermatomycose, y compris l'onychomycose et la dermatophytose
dans lequel
l'oxyde nitrique est chargé dans ledit dispositif de façon à ce que ledit dispositif élue de l'oxyde nitrique (NO) dudit polymère éluant dans une dose thérapeutique lors d'une utilisation au niveau d'un site de dermatomycose sur un corps vers ledit site.

41. Utilisation selon la revendication 40, dans lequel ladite dose thérapeutique se situe entre 0.001 et 5000 ppm, par exemple 0.01 à 3000 ppm, par exemple 0.1 à 1000 ppm, par exemple 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, ou 100 ppm.

42. Polymère éluant l'oxyde nitrique (NO) pour l'utilisation pour le traitement de la dermatomycose, y compris l'onychomycose et la dermatophytose, dans lequel
l'oxyde nitrique est chargé dans un dispositif de façon à ce que ledit dispositif élue de l'oxyde nitrique (NO) dudit polymère éluant dans une dose thérapeutique lors d'une utilisation au niveau d'un site de dermatomycose sur un corps vers ledit site.

43. Polymère éluant l'oxyde nitrique (NO) selon la revendication 42, dans lequel ladite dose thérapeutique se situe entre 0.001 et 5000 ppm, par exemple 0.01 à 3000 ppm, par exemple 0.1 à 1000 ppm, par exemple 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, ou 100 ppm.
